# EUROPEAN PATENT APPLICATION

(11) **EP 4 704 111 A1**
(43) Date of publication of application: **04.03.2026**
(21) Application number: 24199564.6
(22) Date of filing: 10.09.2024
(51) Int. Cl.: G16H 50/20, A61B 10/00

(54) **MENSTRUAL CYCLE ESTIMATION AND USE IN INFECTION PREDICTION**

(30) Priority: 27.08.2024 US 202463687487 P
(71) Applicant: Koninklijke Philips N.V., 5656 AG Eindhoven (NL)
(72) Inventor: GARCIA ARAUJO DA SILVA, Ikaro, Eindhoven (NL); MARIANI, Sara, Eindhoven (NL); SALVATI, Emmanuele, 5656AG Eindhoven (NL); CONROY, Bryan, Eindhoven (NL)
(74) Representative: Philips Intellectual Property & Standards

(57) **Abstract**

A technique for estimating whether or not a subject has an infection from their physiology that is capable of adjusting for menstrual cycle states. The technique comprises obtaining physiological data representing one or more of the subject's physiological parameters at a first point in time, such as skin temperature and resting heart rate. The physiological data is then used to estimate the subject's menstrual state at the first point in time. In particular, the subject's menstrual state is known to impact their physiology. Both the physiological data and the estimated menstrual state are then subsequently used to estimate the subject's infection state. Specifically, the physiological data and the menstrual state are processed using a machine-learning model.

## Description

### FIELD OF THE INVENTION

This invention was made with Government support under contract no. HDTRA121C0006 awarded by Defense Threat Reduction Agency. The Government has certain rights in this invention. The present invention relates to the field of infection prediction, and in particular to methods of adjusting a prediction of an infection state of a subject from menstrual cycle states estimated from physiological data.

### BACKGROUND OF THE INVENTION

Personal wearable devices, such as smartwatches and fitness trackers, are capable of tracking changes in one or more physiological parameters of a person (e.g., skin temperature, heart rate, etc.). Significant changes in such parameters may be linked to the onset of illness/infection, particularly if observed when the person is in a resting state, e.g., sleeping.

However, it has been recognized that similar changes to physiological parameters can also be observed in women who experience menstrual cycles, depending on which phase/state of the menstrual cycle they are currently experiencing. Algorithms aimed at predicting the presence of infection in an individual may thus provide a larger number of false positives and/or false negatives for women due to the increased variation in their physiology.

There is therefore a desire for a method of predicting whether an individual has an infection that can both estimate, and account for, the menstrual state of said individual.

### SUMMARY OF THE INVENTION

The invention is defined by the claims.

According to examples in accordance with an aspect of the invention, there is provided a computer-implemented method of generating infection state information of a subject indicative of whether or not the subject has an infection. In particular, the computer-implemented method takes into account one or more menstrual states of the subject when generating the infection state information. The computer-implemented method comprises obtaining physiological data representing one or more physiological parameters of the subject at a first point in time, processing the physiological data to generate menstrual state information of the subject indicative of at least a menstrual state of the subject at the first point in time, and processing the physiological data and the menstrual state information using a machine-learning model to generate the infection state information.

The present disclosure provides a technique for predicting an infection state of a subject (i.e., whether not the subject has an infection) at a first point in time based on physiological information/data for the subject. Specifically, said physiological data may suggest a deviation of the subject's physiology from a nominal/expected state, indicative of the subject having an infection.

Furthermore, the proposed approach also takes into account a possible menstrual state of the subject, which is known to similarly cause variations in a subject's physiology. In particular, the technique comprises generating menstrual state information (indicative of one or morestates or phases of the subject's menstrual cycle) from the physiological data, and subsequently processing both the physiological data and the menstrual state information to predict the infection state of the subject.

More precisely, the physiological data and the menstrual state information are processed using a machine-learning model that has been trained to generate infection state information indicative of whether or not the subject has an infection. Accordingly, processing the physiological data and the menstrual state information using the machine-learning model may comprise determining an expected value for each of the one or more physiological parameters responsive to the menstrual state information, and processing each expected value and the physiological data using the machine-learning model to generate the infection state information. In particular, the machine-learning model may be capable of identifying deviations of the physiological data from the expected values, indicative of the subject having an infection.

In certain examples, processing the physiological data to generate the menstrual state information may comprise combining the physiological data with historic physiological data to produce a plurality of physiological time-series, producing a plurality of physiological functions by, for each physiological time-series, processing said physiological time-series to determine a respective physiological function, processing the determined physiological functions to generate a fused function representing a combination of the physiological functions, and generating the menstrual state information responsive to one or more morphological features of the fused function.

Specifically, in the above context, each physiological time-series may represent a respective physiological parameter of the subject over a predetermined time interval up to the first point in time. Furthermore, each physiological function may represent an estimate of a respective physiological parameter of the subject over the predetermined time interval. Accordingly, the menstrual state information may indicate different menstrual states of the subject over the predetermined time interval.

It is recognized that the different physiological parameters of the subject may vary (quasi-) periodically due to the menstrual cycle of the subject. Identification and/or usage of information pertaining to said periodicity in the one or more physiological time-series may therefore be used to infer the menstrual cycle, and, thus, the different menstrual states of the subject at different times (e.g., at the first point in time).

The present embodiment thus proposes determining a function for each physiological time-series that estimates the variation in the respective physiological parameter over the time interval. Each function may then be subsequently combined into a "fused" function. Particularly, the fused function may be representative of the variation in physiological parameters across all physiological time-series, thus providing an improved prediction of the (quasi-)periodicity of physiological parameters caused by the menstrual cycle compared to using just a single physiological time-series. Menstrual state information may then be subsequently determined responsive to morphological features in the fused function (e.g., local minima, local maxima, gradients, etc.) that are indicative of the (quasi-)periodicity of the menstrual cycle.

In some examples, processing each physiological time-series to determine a respective physiological function may comprise identifying one or more periodicities present in the physiological time-series, and generating the respective physiological function responsive to the one or more periodicities. For example, the one or more periodicities may be identified by processing the physiological time-series using a spectral analysis technique.

Furthermore, the physiological function may be generated responsive to periodicities of the one or more periodicities within a predefined periodicity range. For example, the predefined periodicity range may correspond to an expected range of periodicities for the menstrual cycle, e.g., a time period between 20 and 36 days.

Furthermore, the physiological function may comprise a plurality of sinusoidal components. In particular, each sinusoidal component may comprise (e.g., be characterized by or represented by) a periodicity corresponding to a respective periodicity of the one or more periodicities, and an amplitude responsive to a degree at which the respective periodicity is present in the physiological time-series.

In some examples, processing the determined physiological functions to generate the fused function may comprise determining a correlation between each physiological function and respective physiological time-series, producing a plurality of weighted physiological functions by, for each physiological function, weighting said physiological function responsive to a respective correlation, and generating the fused function responsive to the plurality of weighted physiological functions.

For example, a particular physiological function may be weighted more heavily if it has a stronger correlation with the corresponding physiological time-series. In particular, a stronger correlation may indicate the function is more representative of the variation in physiological parameter and thus more representative of the (quasi-)periodicity of the menstrual cycle.

Additionally, generating the fused function may further comprise, prior to weighting each physiological function, normalizing each physiological function. Preferably, normalizing a physiological function may comprise subtracting, from the physiological function, a mean of the physiological function, and dividing the physiological function by a standard deviation of the physiological function.

In some examples, generating the menstrual state information may comprise identifying one or more peaks and/or valleys of the fused function, and generating the menstrual state information responsive to the one or more identified peaks and/or valleys.

In particular, each identified peak and/or valley may be indicative of a menstrual state and/or a change in a menstrual state of the subject. For example, a particular menstrual state may be assigned to a time of an identified peak (or valley) and to times within a predefined deviation from the identified peak (or valley).

Additionally, generating the menstrual state information may further comprise determining a menstrual effect strength responsive to a measure of similarity between each physiological function and its respective physiological time-series. Subsequently, the menstrual effect strength may be included in the menstrual state information.

For example, a strong (e.g., larger) measure of similarity may be indicative that the menstrual cycle of the subject has a strong and/or well-defined influence on the physiology of the subject. The determined menstrual effect strength may then be used when generating the infection state information.

In some examples, each physiological time-series may comprise a plurality of daily averages of the respective physiological parameter over a predetermined number of days. Specifically, the plurality of daily averages may be determined responsive to the physiological data and the historic physiological data.

Furthermore, the plurality of daily averages may be determined responsive to a portion of the physiological data and a portion of the historic physiological data measured when the subject is asleep.

In some examples, the one or more physiological parameters of the subject may comprise any one or more of: a skin temperature, a resting heart rate, a heart rate variability, a blood oxygen saturation, a blood pressure, a breathing rate, an electrical activity, a stress level, and/or a sleep quality of the subject.

Also provided is a computer program product comprising computer program code means which, when executed on a computing device having a processing system, cause the processing system to perform all of the steps of any herein proposed method.

Further provided is a system for generating infection state information of a subject indicative of whether or not the subject has an infection. In particular, the system is configured to take into account one or more menstrual states of the subject when generating the infection state information. The system comprises a wearable device configured to be wearable by the subject and, when worn by the subject, acquire physiological data of the subject representing one or more physiological parameters of the subject. The system also comprises a processing system configured to obtain, from the wearable device, physiological data representing one or more physiological parameters of the subject at a first point in time, process the physiological data to generate menstrual state information of the subject indicative of at least a menstrual state of the subject at the first point in time, and process the physiological data and the menstrual state information using a machine-learning model to generate the infection state information.

The system, particularly the processing system, may be adapted to carry out or perform the function of any herein disclosed method, and vice versa.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for generating infection state information of a subject according to a proposed method;
Fig. 2 shows a flowchart illustrating the proposed method;
Fig. 3 shows a flowchart illustrating a step of the proposed method according to an embodiment;
Fig. 4 shows a flowchart illustrating a step of the proposed method according to a further embodiment; and
Fig. 5 illustrates an example of physiological time-series, physiological functions and a fused function.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a technique for generating infection state information regarding a subject. Specifically, the infection state information is (at least) indicative of (or otherwise predictive of) whether or not the subject has an infection.

In order to determine the infection state information, the method comprises first obtaining physiological data from the subject representative of one or more of the subject's physiological parameters (e.g., heart rate, skin temperature, etc.) at a first point in time. Particularly, the physiological data may (at least) be indicative of the subject's infection state (i.e., whether or not the subject has an infection) at the first point in time. The physiological data may be acquired, for example, by one or more devices and/or sensors worn by the subject.

The method further comprises processing the physiological data to generate menstrual state information for the subject. The menstrual state information indicates at least a menstrual state (i.e., stage of the menstrual cycle) of the subject at the first point in time. In particular, it is recognized that the menstrual state of the subject may also impact their physiological parameters. Thus, to generate the infection state information, the method comprises processing both the physiological data and the menstrual state information. More specifically, the physiological data and the menstrual state information are processed using a machine-learning model.

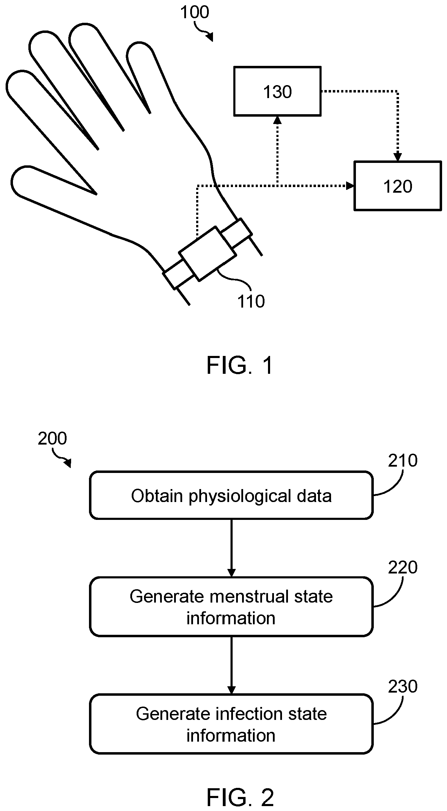

Fig. 1 illustrates a system 100 to which herein proposed approaches may be applied. The system comprises a wearable device 110 and a processing system 120.

The wearable device 110 is configured to be worn by a subject. For example, in Fig. 1, the wearable device 110 is depicted as a wristband or watch that the subject would wear around their wrist. The wearable device 110 may, however, take the form of another wearable object, such as a ring that the subject would wear on their finger, an armband that the subject would wear around their upper arm, or a patch that the subject would attach (e.g., stick) to their skin. Those skilled in the art will be familiar with other forms of wearable devices.

The wearable device 110 is further configured to, when worn by the subject, acquire physiological data of the subject. Specifically, the physiological data comprises data measured by the wearable device 110 relating to (i.e., representative of) one or more physiological parameters of the subject. To this end, the wearable device 110 may comprise one or more sensors or sensing systems for measuring data representing each of the one or more physiological parameters. For example, the wearable device 110 may (preferably) comprise one or more sensors for measuring the subject's skin temperature, the subject's heart rate, and/or the subject's heart rate variability. The one or more sensors may also be capable of measuring the subject's blood oxygen saturation, blood pressure, breathing rate, electrical activity, stress levels, sleep quality, and so on. Those skilled in the art will be familiar with other forms of physiological parameters that may be measured by wearable sensors.

The one or more sensors may utilize any of electrical, optical and/or acoustic techniques (or any combination thereof) for measuring the subject's physiological parameters.

The wearable device 110 may be configured to acquire the physiological data periodically, e.g., at predefined time intervals. For example, the wearable device 110 may acquire the physiological data every second, or every 5 seconds, or every minute, etc. Such a periodicity may be dependent on a sampling rate/speed of the wearable device 110 (i.e., how fast the wearable device 110 can or is configured to acquire discrete measurements) and/or may be reflective of a sampling rate of the one or more sensors.

Accordingly, following from the above, the physiological data may correspond to measurements of the subject's physiological parameters at a particular point in time. In other words, the physiological data may be representative of the one of more physiological parameters at said point in time.

Here, the point in time may refer to a particular moment/instance in time, e.g., 10:55 pm. For example, said moment in time may refer to a timestamp defining a precise time and date. In such cases, the physiological data may correspond to a singular measurement of the one or more physiological parameters acquired at the moment in time.

In other examples, the point in time may refer to a particular time interval, e.g., 10:00 PM to 11:00 PM, or a particular time period, e.g., evening. In such cases, the physiological data may correspond to a plurality of measurements of the one or more physiological parameters acquired over the time interval/period.

Following acquisition of the physiological data, the wearable device 110 may be configured to provide the physiological data to the processing system 120. In particular, the processing system 120 may receive (or otherwise obtain) the physiological data from the wearable device 110 as a (semi-)continuous data stream. Specifically, said data stream may comprise a number of data portions, where each data portion corresponds to physiological data acquired at a particular moment in time. In such cases, the processing system 120 may receive each data portion sequentially, e.g., as set by the sampling periodicity of the wearable device 110.

As another option, the processing system 120 may receive the physiological data as a data chunk comprising physiological data acquired over a time interval, e.g. several minutes, several hours, a day, etc. In relation to later disclosure, the data chunk may be indicative of averages and/or variabilities of the one or more physiological parameters over the time interval.

Following obtaining the physiological data, the processing system 120 is configured to process the physiological data according to a proposed method, as will be later disclosed.

In some examples, the processing system 120 may be arranged as part of (or otherwise be comprised by) the wearable device 110, e.g., an internal processing chip of a smartwatch. In other examples, the processing system 120 may be external to the wearable device 110 and/or greatly distanced from the wearable device 100, e.g., a server owned and/or controlled by a provider of the wearable device. In such cases, the wearable device 110 may communicate with the processing system 120 over a wireless network.

The system 100 may further comprise a memory 130. Accordingly, the wearable device 110 may be configured to (also) provide the physiological data to the memory 130. Put another way, the wearable device 110 may store the physiological data in the memory 130 such that it can be (later) retrieved, e.g., by the processing system 120.

When stored in the memory 130, the physiological data may take the form of (or otherwise be referred to as) historic physiological data, i.e., physiological data acquired at a previous point in time. Accordingly, the historic physiological data may be representative of one or more physiological parameters of the subject at the previous point in time. Furthermore, the processing system 120 may retrieve the historic physiological data from the memory 130, e.g., for the purposes of performing the proposed method.

In some examples, the memory 130 may be arranged as part of (or otherwise be comprised by) the wearable device 110, e.g., an internal memory of a smartwatch. In other examples, the memory 130 may be external to the wearable device 110 and/or greatly distanced from the wearable device 100, e.g., a server owned and/or controlled by a provider of the wearable device. In such cases, the wearable device 110 may communicate with the memory 130 over a wireless network.

Fig. 2 shows a flowchart outlining a proposed method 200 of generating infection state information of a subject. In particular, the method 200 may be performed by a processing system, e.g., the processing system 120.

The method 200 comprises a step 210 of obtaining physiological data representing one or more physiological parameters of the subject. More specifically, the physiological data corresponds to (or comprises) measurements of the subject's physiological parameter(s), e.g., skin temperature, resting heart rate, heart rate variability, etc. Such measurements may be acquired by a wearable device worn by the subject, e.g., the wearable device 110.

Furthermore, the physiological data is representative of the one or more physiological parameters at a first point in time. As discussed previously, the first point in time may correspond to a particular/precise moment in time. Accordingly, said moment in time may also correspond to the time when the physiological data was measured/acquired. In other words, the physiological data may comprise a single measurement of the one or more physiological parameters taken at the first point in time.

Alternatively, the first point in time may correspond to a broader definition of time such as a time interval or time period, e.g., morning, night, daytime, nighttime, a particular day, etc. Accordingly, the physiological data may comprise a plurality of measurements taken at different times during the time interval.

Preferably, the physiological data may be acquired at a time, or over a period of time, when the subject is in a resting state, such as when the subject is asleep. In such a state, the subject's physiology can be expected to be more stable, e.g., compared to if the subject was performing an activity. Accordingly, it can be assumed that the subject's measured physiology may primarily be affected by, e.g., their infection state and/or their menstrual state.

Additionally, in cases where the physiological data comprises a combination of data when the subject is/was asleep and when the subject is/was awake, later steps of the method 200 may only use portions of the physiological data corresponding to when the subject is/was asleep.

The method 200 further comprises a step 220 of processing the physiological data to generate menstrual state information of the subject. The menstrual state information is indicative of at least a menstrual state of the subject at the first point in time. In relation to later disclosure, the menstrual state information may also indicate menstrual states of the subject at further points in time different to the first point in time, and/or parameters associated with the menstrual states indicated by the menstrual state information.

In particular, it is recognized that the subject's physiology at the first point in time (as represented by the physiological data) may be representative of the subject's menstrual state at the first point in time.

More precisely, it is known that a subject's physiology can vary (quasi-) periodically according to their menstrual cycle. Particularly, the ovulation phase of the menstrual cycle is known to be associated with increased skin temperature, increased heart rate, and decreased heart rate variability. Identification of the (quasi-) periodic variation in these physiological parameters can thus be used to estimate the subject's menstrual cycle, and thus their menstrual state at a particular time, e.g., the first point in time.

Accordingly, in some examples, step 220 may comprise comparing and/or combining the physiological data with further physiological data (i.e., physiological data associated with a point in time other than the first point in time) to estimate the subject's menstrual cycle. More precisely, the menstrual cycle may be estimated over a time interval given by the combination of the physiological data and the further physiological data. Furthermore, the menstrual cycle may be estimated from an observed (quasi-) periodicity in the subject's physiological parameters over the time interval. Accordingly, the menstrual state information may be generated by (at least) identifying (from the estimated menstrual cycle) the subject's (estimated) menstrual state at the first point in time.

For the sake of compactness, the prefix "quasi-" will be dropped in the following disclosure. Accordingly, wherever "periodic" is stated, it will be understood to mean either periodic or quasi-periodic.

Further explanation of how to estimate the subject's menstrual cycle and/or menstrual state from physiological data will be later disclosed.

In the present context, the first point in time may refer to a substantially current, present, or recent point in time. For example, the first point in time may refer to a time no more than a day prior to a present time. Accordingly, when estimating the subject's menstrual cycle, the physiological data may be combined with previous (i.e., historic) physiological data, i.e., physiological data acquired at times and/or over a time interval prior to the first point in time. Thus, the menstrual cycle may be estimated over a time interval ending at the first point in time.

Of course, the first point in time may (instead) refer to a (substantially) past/previous point in time. In other words, the menstrual state information (and subsequently the infection state information) may be determined for a point in time in the past. Accordingly, when estimating the subject's menstrual cycle, the physiological data may be combined with further physiological data acquired both before and after the first point in time. Thus, the menstrual cycle may be estimated over a time interval containing the first point in time.

As an alternative technique, step 220 may instead comprise comparing the physiological data against a predetermined model of the menstrual cycle. In particular, the predetermined model may describe expected values, ranges and/or variabilities for different physiological parameters at different stages of the menstrual cycle. The menstrual state at the first point in time may therefore be estimated from the predetermined model, e.g., by identifying the phase in the model that best matches the physiological parameter values given by the physiological data.

Following generation of the menstrual state information, the method 200 comprises a step 230 of processing the physiological data and the menstrual state information to generate the infection state information. Specifically, the physiological data and the menstrual state information are processed using a (trained) machine-learning model.

The machine-learning model is configured to evaluate the physiological data and output an estimation/prediction of the subject's infection state which is included in the infection state information. In particular, the machine-learning model may look for deviations of the subject's physiological parameters away from expected values, ranges and/or trends which the machine-learning model has learned from training. Identification of significant deviations (or at least deviations above a predetermined threshold and/or that satisfy one or more predefined criteria) may prompt the machine-learning model to output an indication that the subject has an infection, e.g., a high percentage probability of the subject having an infection.

Furthermore, the machine-learning model is configured to account for the subject's menstrual state when evaluating the physiological data. More precisely, the machine-learning model takes as input the menstrual state indicated by the menstrual state information from step 220 and adjusts its evaluation procedure accordingly.

In particular, the machine-learning model is trained to recognize that certain menstrual states may impact the subject's physiological parameters, e.g., cause them to deviate from nominal values/ranges. Accordingly, dependent on the provided menstrual state, the machine-learning model may be more lenient when evaluating deviations of the subject's physiological parameters from expected values, and/or require larger deviations in order to output an indication that the subject has an infection.

In some examples, the machine-learning model may adjust its expected values responsive to the provided menstrual state. In other words, the machine-learning model may determine an expected value for each of the one or more physiological parameters represented by the physiological data responsive to the menstrual state information. The machine-learning model may then process the expected value(s) along with the physiological data to generate the infection state information. For example, the machine-learning model may compare each expected value against the physiological data (e.g., to determine a measure of difference and/or deviation) to generate the infection state information.

In other examples, the machine-learning model may first process the physiological data to produce an initial estimate for the subject's infection state. The machine-learning model may then adjust (or otherwise correct) the initial infection state (thereby generating the infection state information) responsive to the subject's menstrual state.

The machine-learning model may also use further information provided by the menstrual state information when evaluating the physiological data and/or generating the infection state information, as will be later disclosed.

Accordingly, the machine-learning model is configured to output infection state information that is representative of both the subject's physiology and their menstrual state at the first point in time. In this way, the infection prediction provided by the method 200 may result in fewer false positives for women compared to an infection prediction method that does not account for the subject's menstrual state.

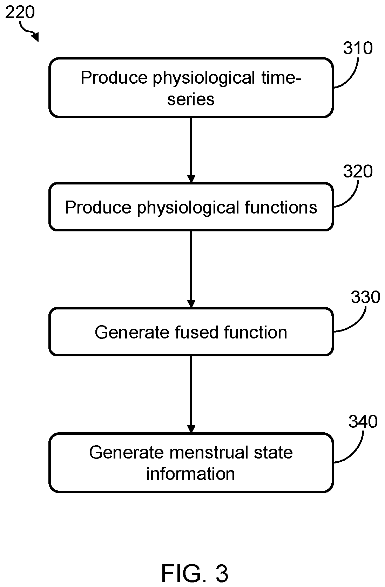

Fig. 3 shows a flowchart outlining an example process by which to generate the menstrual state information from the physiological data, i.e., sub-steps of step 220. Specifically, the process provided in Fig. 3 relates to the previously disclosed technique of generating the menstrual state information by estimating the subject's menstrual cycle over a time interval.

Accordingly, step 220 may first comprise combining the physiological data with historic physiological data, e.g., to produce combined physiological data. Explicitly, the physiological data corresponds to measurements of the subject's physiological parameters at/over the first point in time, while the historic physiology data corresponds to measurements of the subject's physiological parameters at/over a point in time prior to the first point in time. In this context, the first point in time may (preferably) refer to a present or recent point in time.

The historic physiological data may be chosen in such a way that the combined physiological data covers a predetermined time interval ending at the first point in time. For example, the historic physiological data may comprise any (e.g., all) physiological data acquired prior to the first point in time up to a past point in time as given by the predetermined time interval, e.g., the point in time given by subtracting the predetermined time interval from the first point in time.

Preferably, the predetermined time interval may be no less than 36 days. In this way, the combined physiological data may be expected to cover at least one period of the subject's menstrual cycle.

The physiological data and the historic physiological data may be combined as part of a sub-step 310. Subsequently, sub-step 310 may comprise using the combined physiological data to produce a plurality of physiological time-series. Specifically, each physiological time-series represents a respective physiological parameter of the subject over the predetermined time interval. More precisely, each physiological time-series comprises measurements of the respective physiological parameter over the predetermined time interval, as extracted from the combined physiological data. Accordingly, sub-step 310 may comprise producing a physiological time-series for each physiological parameter represented by the combined physiological data.

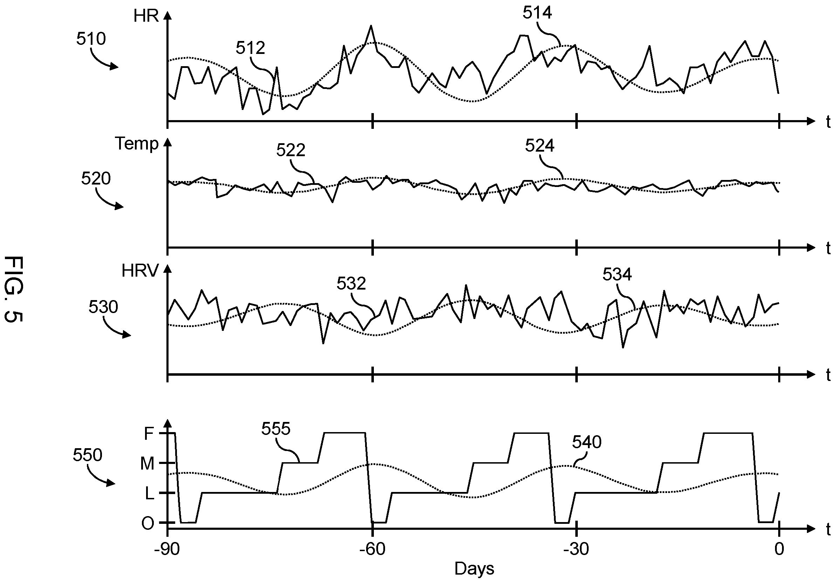

Fig. 5 shows examples of physiological time-series 512, 522 and 532. Specifically, the physiological time-series 512, 522 and 532 are presented as line graphs plotted on graphs 510, 520 and 530, respectively. Each physiological time-series comprises data points representing measurements of a respective physiological parameter at/over an associated point in time, t. In particular, graphs 510, 520 and 530 (and each associated physiological time-series) correspond to the physiological parameters: resting heart rate, HR, skin temperature, Temp, and heart rate variability, HRV, respectively.

Here, heart rate variability may correspond to a determined variance and/or variability in the subject's (resting) heart rate over a corresponding time period. For example, the heart rate variability may be determined using a root mean sequential square difference (RMSSD) method.

In the present examples, each data point corresponds to a daily average of a respective physiological parameter, i.e., an average of the respective physiological parameter over an associated day or part of a day. Accordingly, each daily average may be determined responsive to a portion of the combined physiological data acquired over the associated day. Preferably, the daily average may be determined responsive to a portion of the combined physiological data acquired when the subject is asleep.

Thus, according to the above example, each physiological time-series may comprise a plurality of daily averages of a respective physiological parameter. In particular, the plurality of daily averages may cover a predetermined number of days, e.g., as given by the predetermined time interval.

Of course, each data point may (instead) correspond to a different average, e.g., an hourly average. Alternatively, each data point may correspond to a discrete measurement of the respective physiological parameter at a particular moment in time.

For a sufficiently long predetermined time interval, e.g., over 36 days, each physiological time-series is expected to display periodic variation in the respective physiological parameter due to the subject's menstrual cycle. More precisely, said periodic variation may appear as repeated peaks and valleys/troughs in the values of each respective physiological parameter. Identification of this periodicity can thus be used to estimate the subject's menstrual cycle.

Returning to Fig. 3, step 220 may comprise a sub-step 320 of processing each physiological time-series from sub-step 310 to determine a plurality of respective physiological functions. Specifically, each physiological function (for an associated physiological time-series) represents an estimate of a respective physiological parameter over the predetermined time interval. In other words, each physiological function is an approximation of the associated physiological time-series.

Possible techniques of producing the plurality of physiological functions will be later disclosed.

Preferably, each physiological function is a continuous function that describes the respective physiological parameter as a function of time over the predetermined time interval. Furthermore, to capture the periodic behavior in each physiological time series, each physiological function may be a periodic function and/or comprise periodic components, such as sine and cosine functions/components.

Fig. 5 shows examples of physiological functions 514, 524 and 534, each associated with a respective physiological time-series 512, 522 and 532. The physiological functions 514, 524 and 534 can be seen to approximate the periodicity seen in each physiological time-series 512, 522 and 532, respectively.

Step 220 may further comprise a sub-step 330 of processing the physiological functions from sub-step 320 to generate a fused function. In the present context, the fused function represents a combination of the physiological functions. For example, the fused function may comprise components derived and/or extracted from each of the physiological functions, e.g., a linear or weighted combination of the individual components from each of the physiological functions. Possible techniques of generating the fused function will be later disclosed.

The fused function may thus be representative of the periodicity of each physiological parameter over the predetermined time interval. Put another way, the fused function may represent a unified/combined estimation of the variation in the subject's physiology over the predetermined time interval. Accordingly, the periodicity of the fused function may be used to estimate the subject's menstrual cycle. Particularly, the periodicity of the menstrual cycle is expected to track (or be substantially similar to) the periodicity of the fused function.

As such, step 200 may comprise a (final) sub-step 340 of generating the menstrual state information responsive to the fused function. More precisely, sub-step 340 may comprise generating the menstrual state information responsive to one or more morphological features of the fused function. The morphological features may include any of: the positions and/or values of (local) minima and/or maxima of the fused function, gradients of the fused function, and/or (time) periods of the fused function. In particular, the morphological features may be indicative of particular phases/states and/or phase transitions of the menstrual cycle.

Accordingly, from the morphological features, sub-step 340 may comprise estimating the subject's menstrual cycle. This may comprise estimating the menstrual cycle over the full extent of the predetermined time interval (i.e., for all times covered by the predetermined time interval) or only for certain parts of the predetermined time interval, e.g., parts associated with morphological features of the fused function.

As part of estimating the menstrual cycle, different menstrual states may be assigned to different times over the predetermined time interval, including the first point in time. Accordingly, the menstrual state information generated in sub-step 340 may indicate (e.g., include) these different menstrual states.

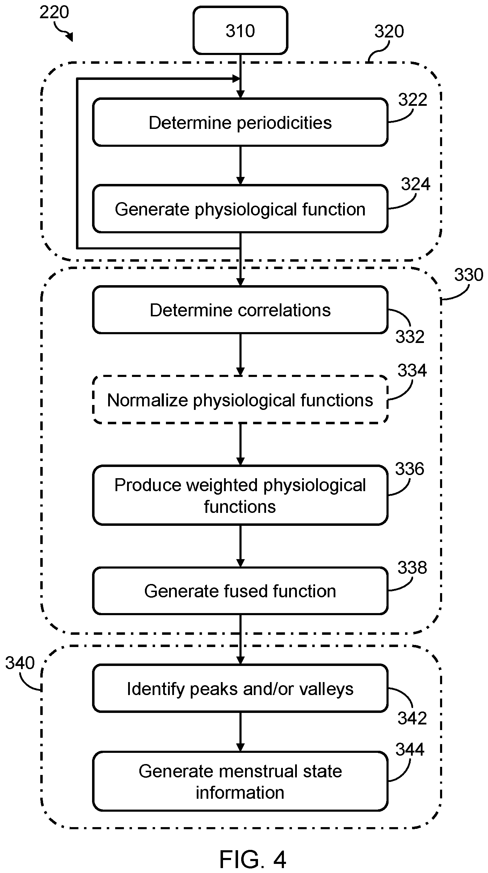

Fig. 4 shows a flowchart outlining a further embodiment of step 220. In particular, Fig. 4 presents potential embodiments of sub-steps 320, 330 and 340 of step 220.

As previously mentioned, sub-step 320 comprises processing each physiological time-series to determine corresponding physiological functions. Preferably, each physiological function approximates (or is otherwise representative of) the periodicity present in its respective physiological time-series. To achieve this, processing of each physiological time-series may comprise a sub-step 322 of identifying (or otherwise extracting) one or more periodicities present in each physiological time-series.

To provide more context, the periodicity present in each physiological time-series may be estimated as a superposition (i.e., linear combination) of periodic (e.g., sinusoidal) functions. Determination of the periodicities of these periodic functions (and their relative strengths) within the physiological time-series may be used to reconstruct (i.e., generate) a function that estimates the present periodicity, e.g., a physiological function. Accordingly, a suitable spectral analysis technique may be applied to each physiological time-series to extract the present periodicities.

A preferred example of a spectral analysis technique may be a Lomb-Scargle technique. Specifically, this comprises fitting sinusoidal functions of different periodicity to the physiological time-series, with a better fit resulting in a larger amplitude assigned to the associated period. The result is a Lomb-Scargle periodogram that describes the relative amplitudes of different sinusoidal functions in the physiological time-series.

Of course, other spectral analysis techniques may be used such as other periodogram methods, wavelet fitting, cosinor fitting, discrete cosine transforms, auto-regression fitting, or others as known to those skilled in the art.

Following identification of the one or more periodicities for a respective physiological time-series, the present embodiment may comprise a further sub-step 324 of generating the physiological function for the respective physiological time-series responsive to the one or more periodicities. Specifically, sub-step 324 map comprise constructing the physiological function from (at least some of) the one or more periodicities.

For example, the physiological function may be constructed such that it comprises a plurality of sinusoidal components. Accordingly, each sinusoidal component may be determined responsive to periodicities of the one or more periodicities. More specifically, each sinusoidal component may be characterized by a respective periodicity of the one or more periodicities. Additionally, each sinusoidal component may be characterized by an amplitude which is determined responsive to (e.g., set equal to) a degree at which the respective periodicity is present in the physiological time-series. For example, the amplitude may be responsive to a relative strength and/or amplitude of the respective periodicity (or more precisely of a periodic function with the respective periodicity) within the physiological time-series.

In some examples, the physiological function may be generated responsive to periodicities of the one or more periodicities that are within a predefined periodicity range. For instance, when comprising a plurality of sinusoidal components, the physiological function may only comprise components with periodicities within the predefined periodicity range.

Particularly, the predefined periodicity range may correspond to possible and/or expected periodicities of the menstrual cycle. For example, the predefined periodicity range may correspond to a time period range of 20 to 36 days, being a range of possible time periods of the menstrual cycle. Accordingly, periodicities identified in the physiological time-series that are outside of the predefined periodicity range (e.g., short periodicities, which may be particularly prevalent in noisy data) may be excluded when generating the physiological function.

In the present embodiment, sub-steps 322 and 324 are performed for each physiological time-series produced in sub-step 310. In some cases, this may comprise looping over sub-steps 322 and 324, e.g., once for each physiological time-series. In other words, both sub-steps 322 and 324 may be performed for a particular physiological time-series before performing them for another (e.g., the next) physiological time-series. In other cases, sub-step 322 may be performed for all physiological time-series before performing sub-step 324.

Following generating a physiological function for each physiological time-series, the physiological functions may be combined into a fused function in accordance with sub-step 330. Typically, this may comprise using a statistical analysis technique to fuse the different functions together based on their statistical relevance.

In the present embodiment, generating the fused function may first comprise a sub-step 332 of determining a correlation (or more precisely a correlation value) between each physiological function and respective physiological time-series. Particularly, each correlation may represent a measure of similarity between the associated physiological function and physiological time-series, i.e., how closely the physiological function matches the behavior/variation exhibited by the physiological time-series.

Each correlation may be determined using a quality/goodness-of-fit, a regression analysis and/or a residual analysis method, such as a chi-squared or sum-of-squares method. Accordingly, a strong correlation (which may be indicated by either a large or small correlation value dependent on the method used) may indicate the physiological function agrees well with the physiological time-series.

Following sub-step 332, the determined correlations may be used in a sub-step 336 to produce a plurality of weighted physiological functions. Specifically, each weighted physiological function may be produced by weighting a physiological function responsive to its respective correlation.

In the present context, weighting (or otherwise applying a weighting to) a physiological function may comprise multiplying the physiological function by the (applied) weighting.

Subsequently, the plurality of weighted physiological functions may be used in a sub-step 338 to generate the fused function. For example, the fused function may be produced from a linear combination (e.g., addition) of the plurality of weighted physiological functions. In such examples, the combination of sub-steps 336 and 338 may be (equivalently) considered as producing the fused function from a weighted average of physiological functions.

Preferably, the weighting applied to each physiological function may be greater for those with a stronger correlation.

To provide more context, a strong correlation between a physiological function and its respective physiological time-series may be expected to occur when the physiological time-series has a well-defined (i.e., obvious) periodicity, e.g., as the present frequencies will be easy to extract and subsequently replicate in the physiological function. In other words, a strong correlation may indicate that the periodicity of the menstrual cycle is strongly apparent in the physiological time-series (and equivalently in the physiological function). Thus, when estimating the menstrual cycle, it may be preferable for physiological time-series/functions with a stronger correlation to contribute more towards said estimation. Accordingly, a larger weighting may be preferably applied to physiological functions with a stronger associated correlation.

In some examples, the weighting applied to a physiological function may be proportional to its associated correlation, e.g., when a larger correlation value indicates a stronger correlation. Conversely, when a smaller correlation value indicates a stronger correlation, each weighting may be proportional to the inverse of the correlation.

In further examples, each weighting may be normalized such that the sum of all weightings is equal to 1.

Prior to producing the plurality of weighted physiological functions, each physiological function may first be normalized in a sub-step 334. Specifically, this may comprise subtracting from each physiological function an associated mean (i.e., the mean value of the physiological function) and, subsequently, dividing each physiological function by an associated standard deviation (i.e., the standard deviation of the physiological function). Accordingly, each normalized physiological function will have a mean equal to zero and a variance equal to 1.

Of course, the fused function may be generated using an alternative statistical analysis technique. Suitable alternatives may include principal component analysis techniques, Bayesian estimation techniques, (weighted) Kalman filtering, and expectation maximization techniques, as well as others known to those skilled in the art.

Fig. 5 shows an example of a fused function 540. Specifically, the fused function 540 comes from the combination of physiological functions 514, 524 and 534.

It should be noted that, in the present example, physiological function 534 shows approximately the opposite behavior of physiological functions 514 and 524, i.e., peaks of physiological function 534 approximately coincide with valleys of physiological functions 514 and 524, and vice versa. This is because, at particular phases of the menstrual cycle, e.g. the ovulation phase, increases in skin temperature and resting heart rate are expected to coincide with decreases in heart rate variability. Accordingly, to account for this difference in behavior, the fused function 540 may be produced using a modified (e.g., transformed) form of physiological function 534, e.g., a reflection in the x-axis, such that peaks of physiological function 534 approximately coincide with peaks of physiological functions 514 and 524 (and similarly for valleys).

Returning to Fig. 4, the fused function generated from sub-step 330 may be subsequently used to generate the menstrual state information (e.g., in sub-step 340). This may comprise a sub-step 342 of identifying one or more peaks (i.e., local maximums) and/or valleys (i.e., local minimums) of the fused function. Particularly, it is recognized that such peaks and/or valleys may be indicative of particular menstrual states and/or changes/transitions between menstrual states.

Sub-step 342 may comprise using a peak and/or valley detection algorithm to identify the peaks and/or valleys present in the fused function. Such algorithms may make use of suitable thresholds and/or moving windows to assess the position/location of peaks and/or valleys in the fused function. In further examples, sub-step 342 may comprise taking a derivative of the fused function to identify standing/turning points of the fused function (e.g., from when the derivative is equal to zero). Accordingly, the positions of said standing points may correspond to the positions of peaks and/or valleys of the fused function.

Consequently, the output of sub-step 342 may comprise (time) positions associated with each peak and/or valley, e.g., centers of each peak and/or valley. Sub-step 342 may also output a corresponding value of each peak and/or valley at the associated positions.

Following sub-step 342, the identified peaks and/or valleys may be used in a sub-step 344 to generate the menstrual state information. For example, each peak and/or valley may be used to assign one or more menstrual states to particular times or time periods of the predetermined time interval.

A non-limiting example of the above process will now be explained with reference to Fig. 5. Specifically, a series of menstrual states will be determined responsive to the fused function 540.

As displayed in Fig. 5, the fused function 540 is overlayed on a graph 550 that plots menstrual states on the y-axis against time, t, on the x-axis. In the present example, the fused function is used to assign menstrual states to different times responsive to the (time) positions of peaks and valleys of the fused function. In other words, the values/magnitudes of the peaks and valleys are not used. In other examples, however, said values may be used to determine different menstrual states or assign further information to the menstrual states.

The menstrual cycle can be segmented into four distinct phases/states: menstruation, M, the follicular phase, F, ovulation, O, and the luteal phase, L, which are marked on the y-axis of graph 550. The time-dependency of these states over the predetermined time interval is denoted by the line 555 that indicates which menstrual states the subject may have been in at different parts of the predetermined time interval. The line 555 may be determined responsive to the fused function 540 according to the following.

Peaks of the fused function 540 correspond approximately to peaks in the subject's skin temperature and resting heart rate (and valleys in the subject's heart rate variability) which are expected to coincide with ovulation. Accordingly, the ovulation state may be assigned to times at, and in the vicinity of, peaks of the fused function 540. For example, in Fig. 5, the ovulation state is assigned to a 3-day period centered on the position of each peak of the fused function 540.

Conversely, valleys of the fused function 540 are expected to coincide with menstruation. Particularly, valleys (corresponding to points where the fused function 540 begins to increase) are expected to indicate the beginning of menstruation. Accordingly, the menstruation state may be assigned to times at, and sometime after, valleys of the fused function 540. For example, in Fig. 5, the menstruation state is assigned to a 6-day period beginning at the position of each valley of the fused function 540.

The follicular phase occurs between menstruation and ovulation. Accordingly, times from a valley to a peak of the fused function 540 that are not assigned to the ovulation state or the menstruation state may be assigned to the follicular phase.

The luteal phase occurs between ovulation and menstruation. Accordingly, times from a peak to a valley of the fused function 540 that are not assigned to the ovulation state or the menstruation state may be assigned to the luteal phase.

The resulting line 555 tracks these estimated menstrual states over the predetermined time interval. In particular, transitions between different menstrual states can be seen, in addition to several full menstrual cycles.

The menstrual states determined according to the above process may then be included in the menstrual state information. For example, the menstrual state information (generated according to sub-step 344) may include, or otherwise be indicative of, the line 555. The menstrual state information may then be used according to the method 200 in Fig. 2 to generate infection state information of the subject.

In some cases, as stated previously, the menstrual state information may include (or otherwise be indicative of) further information. Particularly, the menstrual state information may be further indicative of one or more parameters (e.g., quality) associated with the menstrual states indicted by the menstrual state information. Accordingly, step 220 of the method 200 may comprise generating this further information and including it in the menstrual state information.

In some examples, step 220 may comprise determining a menstrual effect strength associated with the determined menstrual states. Specifically, the menstrual effect strength may be indicative of how strongly the subject's menstrual states/cycle affects their physiology. Said strength may be evaluated from how pronounced the variability and/or consistency in the subject's physiological parameters is. Accordingly, the menstrual effect strength may be determined responsive to the physiological time-series.

In some examples, a menstrual effect strength may be determined for the menstrual cycle as whole. In other examples, a menstrual effect strength may be determined just for the menstrual state assigned to the first point in time, or for all menstrual states determined over the predetermined time interval.

The menstrual effect strength(s) may take into account a combined variability in the subject's physiological parameters across all physiological time-series. For example, the menstrual effect strength(s) may be determined responsive to a measure of similarity between each physiological time-series and its respective physiological function. In this context, the measure of similarity may correspond to how closely the physiological function matches the physiological time-series, either as a whole (similar to the previously disclosed correlations) or at particular times of the predetermined time interval.

To provide more context, each physiological function may represent an estimated (and thus expected) variation in an associated physiological parameter over the predetermined time interval. Accordingly, a large difference between the phycological function and the respective physiological time-series (and thus a weak measure of similarity) at a particular point in time may indicate that the menstrual state at that point in time does/did not have a strong effect on the subject's physiology.

The menstrual effect strength(s) may be determined from a combination (e.g., sum, average, etc.) of the measures of similarity determined for each physiological function and physiological time-series pair. Subsequently, the menstrual effect strength(s) may be included in the menstrual state information and assigned to respective menstrual states.

The menstrual effect strength(s) may be used, e.g., by the machine-learning model in step 230 of the method 200 when generating the infection state information. For example, the menstrual effect strength(s) may be used to determine how much weight/influence to associate with the menstrual state(s) when estimating the infection state of the subject from their physiological data.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method (200) of generating infection state information of a subject indicative of whether or not the subject has an infection, wherein said computer-implemented method takes into account one or more menstrual states of the subject, the computer-implemented method comprising:
obtaining physiological data representing one or more physiological parameters of the subject at a first point in time (210);
processing the physiological data to generate menstrual state information of the subject indicative of at least a menstrual state of the subject at the first point in time (220); and
processing the physiological data and the menstrual state information using a machine-learning model to generate the infection state information (230).

2. The computer implemented method of claim 1, wherein processing the physiological data and the menstrual state information using the machine-learning model comprises:
determining an expected value for each of the one or more physiological parameters responsive to the menstrual state information; and
processing each expected value and the physiological data using the machine-learning model to generate the infection state information.

3. The computer-implemented method of any one of claims 1 or 2, wherein processing the physiological data to generate the menstrual state information comprises:
combining the physiological data with historic physiological data to produce a plurality of physiological time-series (310), wherein each physiological time-series (512, 522, 532) represents a respective physiological parameter of the subject over a predetermined time interval up to the first point in time;
producing a plurality of physiological functions by, for each physiological time-series, processing said physiological time-series to determine a respective physiological function (320), wherein each physiological function (514, 524, 534) represents an estimate of a respective physiological parameter of the subject over the predetermined time interval;
processing the determined physiological functions to generate a fused function (540) representing a combination of the physiological functions (330); and
generating the menstrual state information responsive to one or more morphological features of the fused function (340), wherein the menstrual state information indicates different menstrual states of the subject over the predetermined time interval.

4. The computer-implemented method of claim 3, wherein, for each physiological time-series, processing said physiological time-series comprises:
identifying one or more periodicities present in the physiological time-series by processing the physiological time-series (322); and
generating the respective physiological function responsive to the one or more periodicities (324).

5. The computer-implemented method of claim 4, wherein the physiological function is generated responsive to periodicities of the one or more periodicities within a predefined periodicity range.

6. The computer-implemented method of any one of claims 4 or 5, wherein the physiological function comprises one or more sinusoidal components, wherein each sinusoidal component comprises:
a periodicity corresponding to a respective periodicity of the one or more periodicities; and
an amplitude responsive to a degree at which the respective periodicity is present in the physiological time-series.

7. The computer-implemented method of any one of claims 3 to 6, wherein processing the determined physiological functions to generate the fused function comprises:
determining a correlation between each physiological function and respective physiological time-series (332);
producing a plurality of weighted physiological functions by, for each physiological function, weighting said physiological function responsive to a respective correlation (336); and
generating the fused function responsive to the plurality of weighted physiological functions (338).

8. The computer-implemented method of claim 7, wherein generating the fused function further comprises, prior to weighting each physiological function, normalizing each physiological function (334), preferably wherein normalizing a physiological function comprises:
subtracting, from the physiological function, a mean of the physiological function; and
dividing the physiological function by a standard deviation of the physiological function.

9. The computer-implemented method of any one of claims 3 to 8, wherein generating the menstrual state information comprises:
identifying one or more peaks and/or valleys of the fused function (342); and
generating the menstrual state information responsive to the one or more identified peaks and/or valleys (344), wherein each identified peak and/or valley is indicative of a menstrual state and/or a change in a menstrual state of the subject.

10. The computer-implemented method of any one of claims 3 to 9, wherein generating the menstrual state information further comprises:
determining a menstrual effect strength responsive to a measure of similarity between each physiological function and its respective physiological time-series; and
including the menstrual effect strength in the menstrual state information.

11. The computer-implemented method of any one of claims 3 to 10, wherein each physiological time-series comprises a plurality of daily averages of the respective physiological parameter over a predetermined number of days, wherein the plurality of daily averages is determined responsive to the physiological data and the historic physiological data.

12. The computer-implemented method of claim 11, wherein the plurality of daily averages is determined responsive to a portion of the physiological data and a portion of the historic physiological data measured when the subject is asleep.

13. The computer-implemented method of any one of claims 1 to 12, wherein the one or more physiological parameters of the subject comprise any one or more of: a skin temperature, a resting heart rate, a heart rate variability, a blood oxygen saturation, a blood pressure, a breathing rate, an electrical activity, a stress level, and/or a sleep quality of the subject.

14. A computer program product comprising computer program code means which, when executed on a computing device having a processing system (120), cause the processing system to perform all of the steps according to any one of claims 1 to 13.

15. A system (100) for generating infection state information of a subject indicative of whether or not the subject has an infection, wherein said system is configured to take into account one or more menstrual states of the subject, the system comprising:
a wearable device (110) configured to:
be wearable by the subject; and
when worn by the subject, acquire physiological data of the subject representing one or more physiological parameters of the subject; and
a processing system (120) configured to:
obtain, from the wearable device, physiological data representing one or more physiological parameters of the subject at a first point in time;
process the physiological data to generate menstrual state information of the subject indicative of at least a menstrual state of the subject at the first point in time; and
process the physiological data and the menstrual state information using a machine-learning model to generate the infection state information.
